# EUROPEAN PATENT SPECIFICATION

(11) **EP 2 841 143 B1**
(45) Date of publication and mention of the grant of the patent: **01.06.2016**
(21) Application number: 13724435.6
(22) Date of filing: 25.04.2013
(51) Int. Cl.: A61M 1/36, B05B 11/04, A61M 3/02, A61M 5/145, A61M 5/148

(54) **FLUID DELIVERY DEVICE HAVING A CONNECTOR AND A COLLAPSIBLE RESERVOIR**
FLÜSSIGKEITSABGABEVORRICHTUNG MIT VERBINDER UND EINEM FALTBAREN RESERVOIR
DISPOSITIF DE DISTRIBUTION DE FLUIDE POURVU D'UN CONNECTEUR ET D'UN RÉSERVOIR COMPRESSIBLE

(30) Priority: 27.04.2012 US 201261639438 P
(43) Date of publication of application: 04.03.2015
(73) Proprietor: Covidien LP, Mansfield, MA 02048 (US)
(72) Inventor: TREMBLAY, Kathleen, Westfield, MA 01085 (US); JONES, Scott, M., University City, MO 63130 (US); STEUBE, Gregory, A., St. Charles, MO 63301 (US); EARHART, Stephen, B., St. Louis, MO 63126 (US); MARTZ, Kevin, R., Desoto, MO 63020 (US); BAKER, Mark, Union, MO 63084 (US)
(74) Representative: Gray, James
(86) International application number: PCT/US2013/038097
(87) International publication number: WO 2013/163364

(56) References cited:
- EP-A2- 1 236 516
- WO-A1-81/02678
- WO-A1-85/03491
- WO-A1-94/19252
- WO-A1-97/35628
- WO-A1-02/089883
- WO-A1-2007/095768
- WO-A1-2009/073482
- WO-A1-2010/086273
- WO-A2-2012/051093
- GB-A- 2 387 589
- US-A- 3 223 289
- US-A- 3 248 012
- US-A- 3 780 732
- US-A- 3 938 514
- US-A- 4 020 978
- US-A- 4 131 217
- US-A- 4 168 032
- US-A- 4 457 455
- US-A- 4 778 082
- US-A- 5 222 629
- US-A- 5 342 313
- US-A- 5 765 708
- US-A- 5 797 881
- US-A- 5 857 593
- US-A- 5 941 426
- US-A- 6 021 930
- US-A- 6 153 238
- US-A1- 2004 035 885
- US-A1- 2004 078 024
- US-A1- 2005 000 514
- US-A1- 2009 057 347
- US-A1- 2010 095 957
- US-A1- 2011 196 304
- US-A1- 2012 090 603
- US-A1- 2012 279 995
- US-B1- 6 315 761
- US-B1- 6 364 163

## Description

### Technical Field

The present disclosure relates to fluid delivery devices and, more particularly, to fluid delivery devices for delivering a fluids medium to a medical device, e.g., vascular access device.

### BACKGROUND

Fluid delivery devices for delivering fluid to a medical device for a variety of reasons are well known. For example, syringes are often used to deliver a variety of fluids to a medical vascular access device to provide medication or flush solutions to the vascular access device. Typically, a syringe is disposable and includes a syringe body, a syringe plunger and a sealing member supported on the plunger. The syringe assembly is unnecessarily complex and expensive to manufacture.
US3938514 discloses a bladder wash method apparatus, WO2009/07348 discloses a metered drop push button dispenser system, and US6315761 discloses an injection device with bellowed reservoir.

### SUMMARY

In one aspect, a fluid delivery device includes a body defining a fluid reservoir and a fluid outlet. A connector includes structure to connect the fluid delivery device to a medical device. The fluid delivery device includes a housing configured to receive the body. The body includes one or more pleats and is collapsible to dispense fluid from the fluid reservoir through the fluid outlet. The fluid delivery device includes a disc supported on the body within the housing. The disc has one or more teeth which engage notches on the housing to lock the body in one of a plurality of positions.

In some embodiments, each of the one or more pleats contains a predetermined volume of fluid.

In some embodiments, the housing defines a cutout dimensioned to receive a clinician's finger to facilitate compression of the body.

In certain embodiments, the connector includes spikes positioned to puncture the body.

In some embodiments, the housing includes markings to identify the volume of fluid dispensed from the reservoir.

In another aspect not of the present invention, the fluid delivery device includes a collapsible body defining a fluid reservoir, a connector including structure to connect the collapsible body to a medical device, and a plurality of levers pivotally attached to the collapsible body. The levers are movable in relation to each other to move the collapsible body from an expanded position to a collapsed position.

In some examples, a catch is configured to hold the levers in a position to retain the body in the collapsed position.

In certain examples, the fluid delivery device includes a latching mechanism including a first arm extending from one of the plurality of levers having at least one tooth and a second arm extending from another one of the plurality of levers including a plurality of notches. The at least one tooth is advanceable over the notches to progressively lock the levers in relation to each other to prevent expansion of the collapsible body.

In another aspect not of the present invention, the fluid delivery device includes a collapsible body defining a fluid reservoir, a connector including structure to connect the collapsible body to a medical device" and a clip positioned about the collapsible body. The clip is movable along the collapsible body towards the connector to dispense fluid from the fluid reservoir.

In some examples, the clip is slidable along the collapsible body.

In certain examples, the collapsible body includes an elongated neck portion and the clip is slidably disposed about the elongated neck portion. The clip can be configured to expand outwardly when the collapsible body is compressed to allow fluid to be dispensed from the fluid reservoir and return to an unexpanded state after compression to prevent reflux into the fluid reservoir.

In yet another aspect not of the present invention, the fluid delivery device includes a collapsible body defining a fluid reservoir, a plunger, and a housing including a connector configured to engage a medical device. The housing is dimensioned to receive the collapsible body and accept the plunger and includes a puncturing port that is configured to puncture the collapsible body when the plunger is advanced to compress the collapsible body.

In some examples, the plunger includes markings to indicate the amount of fluid dispensed from the fluid reservoir.

In yet another aspect not of the present invention, the fluid delivery device includes a deformable body defining a fluid reservoir, an airbag surrounding the deformable body, and a connector configured to connect the fluid delivery device to a medical device, wherein the airbag can be selectively inflated to dispense fluid from the deformable body.

### BRIEF DESCRIPTION OF THE DRAWINGS

FIG. 1A is a side cross-sectional view of a fluid delivery device in an expanded state.
FIG. 1B is a side perspective view of the fluid delivery device of FIG. 1A.
FIG. 1C is a side perspective view of the fluid delivery device of FIG. 1A in collapsed state.
FIG. 2A is a side cross-sectional view of another embodiment of the fluid delivery device in an expanded state.
FIG. 2B is a side cross-sectional view of the fluid delivery device of FIG. 2A in the collapsed state.
FIG. 3 is a side cross-sectional view of another embodiment of the fluid delivery device.
FIG. 4 is side cross-sectional view of another embodiment of the fluid delivery device in an expanded state.
FIG. 5 is a front view of another embodiment of the fluid delivery device.
FIG. 6 is a cross-sectional view of the fluid delivery device of FIG. 5.
FIG. 7 is a front view of another embodiment of the fluid delivery device.
FIG. 8 is a front view of another embodiment of the fluid delivery device.
FIG. 9 is a front view of another embodiment of the fluid delivery device.
FIG. 10 is a cross-sectional view of another embodiment of the fluid delivery device.
FIG. 11 is a front view of another embodiment of the fluid delivery device.
FIG. 12 is a cross-sectional view of another embodiment of the fluid delivery device.
FIG. 13 is a side view of another embodiment of the fluid delivery device.
FIG. 14 is a cross-sectional view of another embodiment of the fluid delivery device.
FIG. 15A is a front perspective view of another embodiment of the fluid delivery device.
FIG 15B is a front perspective view of the fluid delivery device of FIG. 15A with parts separated.
FIG. 15C is a cross-sectional view of the fluid delivery device of FIG. 15A.
FIG. 16 is a front view of another embodiment of the fluid delivery device.
FIG. 17 is a front view of another embodiment of the fluid delivery device.
FIG. 18A is a front perspective view of another embodiment of the fluid delivery device.
FIG. 18B is a cross-sectional view of the fluid delivery device of FIG. 18A.
FIG. 18C is a cross-sectional view of the fluid delivery device of FIG. 18B including a plunger.
FIG. 19 is a cross-sectional view of another embodiment of the fluid delivery device.

### DETAILED DESCRIPTION OF EMBODIMENTS

Embodiments of the presently disclosed fluid delivery device will now be described in detail with reference to the drawings wherein like reference numerals designate identical or corresponding elements in each of the several views. In this description, the term "proximal" is generally used to refer to the portion of the device that is closer to a clinician, while the term "distal" is generally used to refer to the portion of the device that is farther from the clinician.

Many of the containers or fluid delivery devices herein are described as including a collapsible body. As used herein, the collapsible body may be formed of both resilient and non-resilient materials, including but not limited to, polyolefins such as polyethylene and polypropylene, fluorinated polyolefins such as polytetrafluoroethylene and perfluoroalkoxy, fluoroelastomers, polyamides such as nylon, polyesters, acrylic polymers, vinyl halide polymers such as polyvinyl chloride, and copolymers and combinations thereof. In addition, the containers described herein may be formed using any of a variety of known processes or combinations thereof, including injection molding and blow molding.

As used herein, the term connector includes any known structure for connecting the delivery device to another medical device including luer connectors, screw threads, bayonet type couplings and other known connecting structure. Each connector defines a fluid outlet for dispensing a fluid.

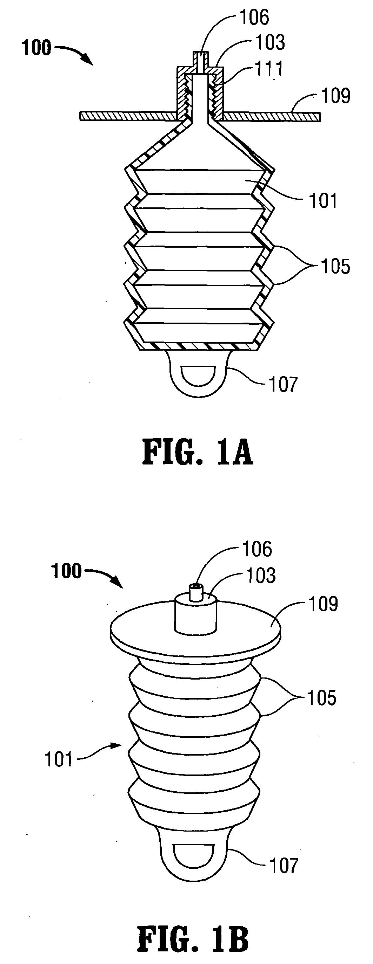

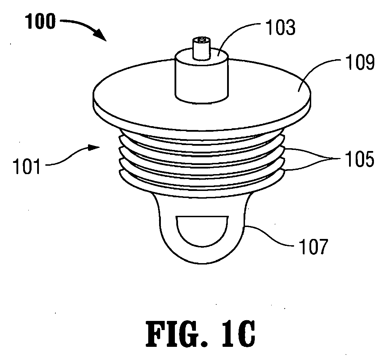

Fig. 1A is a side cross-sectional view and Figs. 1B-1C are perspective views of an embodiment of the presently disclosed fluid delivery device shown generally as 100. Delivery device 100 includes a body 104 that defines a fluid reservoir 101 and includes a connector 103. The connector 103 defines a fluid outlet 103a. The body 104 includes one or more pleats or bellows 105 that can collapse to dispense the fluid contents of the fluid reservoir 101 from a fluid outlet 106 or be expanded to aspirate fluid into the reservoir 101. Each bellow 105 may be dimensioned to contain a predetermined volume of liquid so as to provide a visual indication of the amount of fluid contained in the fluid reservoir 101 and the amount of fluid dispensed from the fluid reservoir 101. For example, each bellow 105 may define a volume of 1mL of fluid. Delivery device 100 may further include a finger loop 107 and a grip flange 109 for grasping the delivery device 100 so that the fluid reservoir 101 may be compressed to dispense fluid from within the fluid reservoir 101. Finger loop 107 and/or grip flange 109 may be integrally formed with body 104, or, in the alternative, may be connected to body 104 by, for example, screw threads.

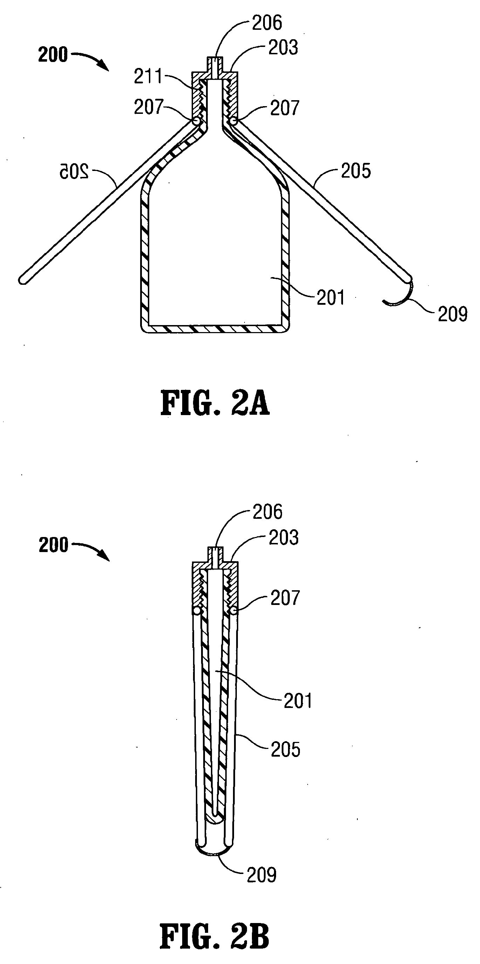

Figs. 2A and 2B are side, cross-sectional views of another embodiment of the delivery device, shown generally as 200. Delivery device 200 includes a body 204 that defines a fluid reservoir 201 and further includes connector 203 defining a fluid outlet 206. The connector 203 is supported adjacent to a distal end of body 204. Two or more levers 205 are pivotally mounted to the distal end of body 203 via hinges 207. The levers 205 are pivotable towards each other about hinges 207 to forcibly collapse the body 204 and dispense the fluid contents of the fluid reservoir 201. In one embodiment, the levers 205 are substantially rigid and can be made of any suitable rigid material. Alternatively, levers 205 may be formed from any material capable of compressing body 204 to dispense the liquid within reservoir 201. In another embodiment, the levers 205 are pivotally supported at the proximal end of body 204 by hinges 205. The hinges 205 may be mechanical or living hinges. In one embodiment, the body 204 is formed from a collapsible non-resilient material including plastics, polymers or the like. Alternatively, resilient, collapsible materials may also be used to form the body 204.

The body 204 of the delivery device 200 may be squeezed using levers 205 from a first expanded position, as shown in Fig. 2A, to a second collapsed position, as shown in Fig. 2B. The fluid reservoir 201 may contain a predetermined amount of fluid that corresponds to a single dose of a medication or flushing liquid. A catch 209 may be provided on one lever 205 which is positioned to engage the other lever 205 to hold the levers 205 in the collapsed position after a predetermined dose of fluid has been dispensed to prevent the body 204 from expanding and generating reflux into the fluid reservoir 201.

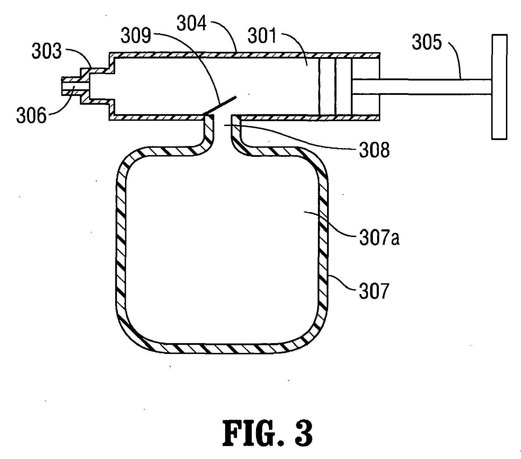

Fig. 3 is a side, cross-sectional view of another embodiment of the delivery device, shown generally as 300. Delivery device 300 includes a substantially rigid body 304 that defines a fluid reservoir 301 and a connector 303 defining a fluid outlet 306. The body 304 is attached to a refill container 307 which defines a receptacle 307a. The body 304 and/or the refill container 307 may be marked with graduations or markings to identify the fluid volume contained within the body 304 and/or container 307. Refill container 307 may be formed of any suitable material which may be a rigid material or a deformable or collapsible material such as a plastic fluid filled bag. The receptacle 307a of refill container 307 communicates with the reservoir 301 of body 304 via an aspiration port 308 and a check valve 309. Check valve 309 is configured to only permit flow from the receptacle 307a into the reservoir 301. In one embodiment, a plunger 305 may be pushed distally within reservoir 301 to dispense fluid from the fluid reservoir 301. The check valve 309 prevents fluid flow into the refill container 307 from the reservoir 301. In addition, the plunger 305 may be pulled proximally to open the check valve 309 to aspirate fluid from the receptacle 307a of refill container 307 into the fluid reservoir 301 through aspiration port 308. In some embodiments, the plunger 305 may be designed to automatically retract to refill the fluid reservoir 301 using any suitable means including, but not limited to, a spring or other biasing device. Further, the area proximal of the plunger 305 may be sealed to allow a negative pressure to build proximally of the plunger 305 as the plunger 305 is pushed distally so that when the plunger 305 is no longer being forced distally, the negative pressure created proximally of the plunger 305 pulls the plunger 305 back in the proximal direction. In one embodiment, the container 307 can be upended to dump fluid from receptacle 307a into reservoir 301 through aspiration port 306.

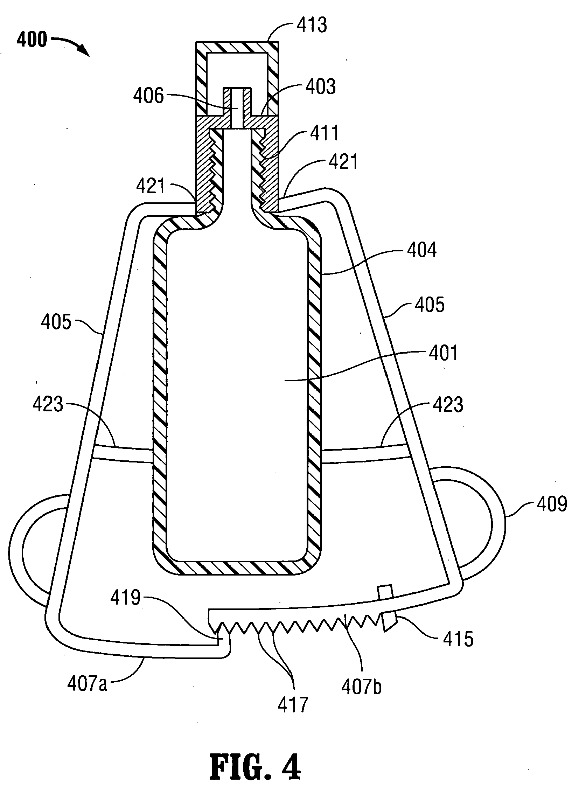

Fig. 4 is a side, cross-sectional view of an embodiment of the delivery device, shown generally as 400. Delivery device 400 includes a collapsible body 404 that defines a fluid reservoir 401 and includes a connector 403. Two or more levers 405 are pivotally secured to body 404 adjacent luer 403. The levers 405 are movable toward each other to forcibly compress and collapse the body 404 to dispense the fluid contents of the fluid reservoir 401. The levers 405 may be made of any suitable rigid material. The hinges 421 may be mechanical or living hinges, and are shown in Fig. 4 as a living hinge.

Levers 405 include a latching mechanism comprising a first arm 407a having at least one tooth 419 that interfaces with notches 417 that are disposed on second arm 407b. Each notch 417 may be designed to correspond to a predetermined amount of fluid being dispensed out of the delivery device 400. For example, each notch may correspond to 1mL of dispensed fluid. Delivery device 400 may further include finger loops 409 for gripping levers 405 and a cap 413 for sealing or covering connector 403. The delivery device 400 can also include a member, such as one or more tethers 423, that attach the outside of the body 404 to an inner surface of the levers 405 such that when the levers 405 are pulled apart, the body 404 and fluid reservoir 401 defined therein will expand to allow fluid to be drawn into the fluid reservoir 401.

In use, the levers 405 are pushed together to dispense fluid from reservoir 401. As this occurs, the tooth 419 of the latching mechanism is advanced over the notches 417 to progressively lock the levers 405 to different degrees of closure. Thus, the latching mechanism prevents body 404 from expanding to prevent reflux into reservoir 401. As discussed above, body 401 may be formed from a collapsible resilient or non-resilient material. Latching mechanism prevents reflux caused by the inherent ability of a resilient material to return the body 401 to its non-deformed configuration.

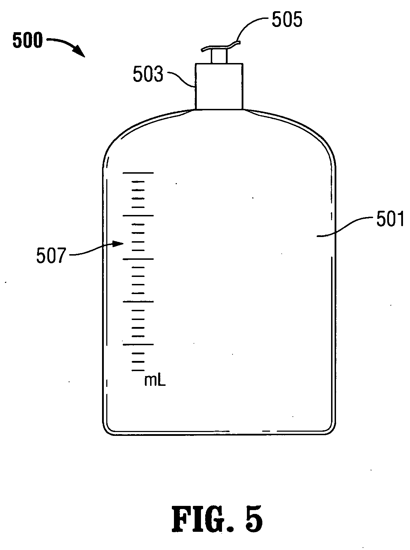

Fig. 5 illustrates an embodiment of the delivery device, shown generally as 500. Delivery device 500 includes a collapsible body 504 that defines a fluid reservoir 501 and a connector 503 formed integrally w/ith body 504. The body 504 is comprised of a flexible plastic bag that may be compressed, rolled from an end in tooth paste dispenser style, or otherwise pressurized to dispense fluid from reservoir 501. The connector 503 may be sealed with a pull tab seal 505 that can be removed when it is desired to use the delivery device 500. The body 504 may have markings 507 to indicate the amount of fluid contained in the fluid reservoir 501.

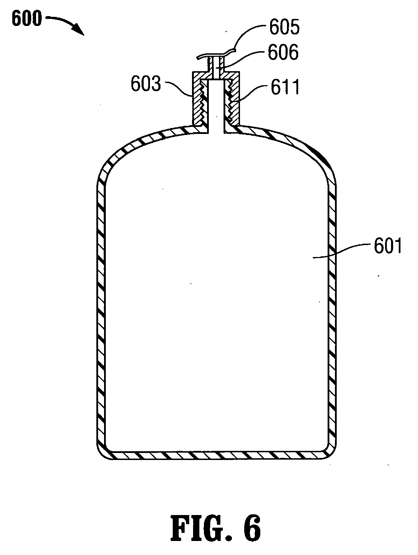

Fig. 6 shows a side, cross-sectional view of an embodiment of a delivery device 600, similar to the embodiment of Fig. 5, including a collapsible body 604 that defines a fluid reservoir 601, a connector 603 and a seal 605 that can be removed when it is desired to use the delivery device 600. The connector 603 defines a fluid outlet 606. Fig.6 is similar to Fig. 5 except that connector 603 of body 604 is a separate member which is threadably attached to body 604.

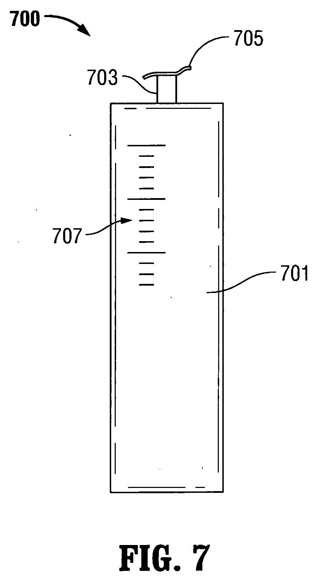

Fig. 7 illustrates an embodiment of a delivery device 700 having a body 704 that defines a fluid reservoir 701, a connector 703 and a seal 705 that can be removed when it is desired to use the delivery device 700. The embodiment of Fig. 7 is similar to the embodiment of Fig. 5 except that the body 704 is elongated is formed from a squeeze-pop type bag. The bag edges of body 704 may be sealed together using RF energy.

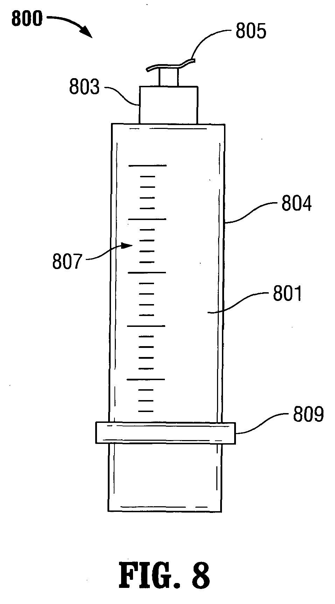

Fig. 8 illustrates an embodiment of the delivery device, shown generally as 800. Delivery device 800 includes a collapsible body 804 that defines a fluid reservoir 801 and a connector 803. The body 804 is comprised of a flexible plastic bag that may be compressed, rolled from an end in tooth paste dispenser style, or otherwise pressurized to dispense fluid. The connector 803 may be sealed with a pull tab seal 805 that can be removed when it is desired to use the delivery device. The body may have markings 807 to indicate the amount of fluid contained in the fluid reservoir 801. The delivery device 800 may further comprise a clip 809 which is positioned about body 804. The clip 809 may be removed from body 804 to disperse fluid from reservoir 801, slid down along body 804 to disperse fluid, or slid upward about body 804 to check the body 804 for patency. The clip 809 may be of any suitable size or shape and may, alternately, roll along or roll up the body 804. For example, the clip 809 may comprise two rollers that sandwich the body 804 under a bias. The rollers may be configured to rotate as the clip 809 is pushed or pulled over the body 804.

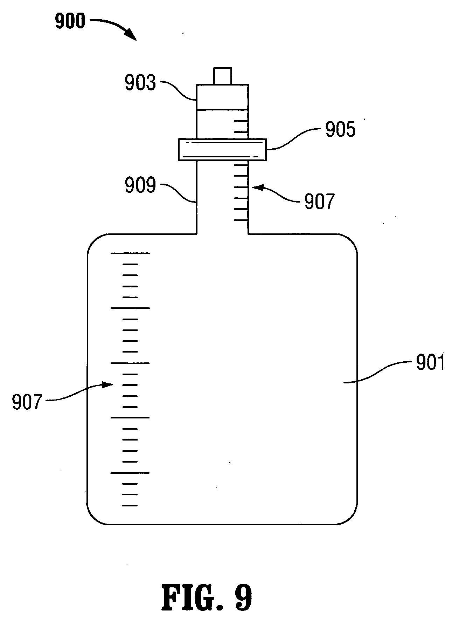

Fig. 9 illustrates another embodiment of the delivery device, shown generally as 900. Delivery device 900 includes a collapsible body 904 that defines a fluid reservoir 901 with an elongated neck portion 909 and a connector 903. The body 904 is comprised of a flexible plastic bag that may be compressed by hand. The body 904 may have markings 907 to indicate the amount of fluid contained in the fluid reservoir 901. The delivery device 900 may further comprise a clip 905 disposed on the elongated neck portion 909 of body 904. The clip 905 may be configured to expand outwardly when body 904 is compressed to allow fluid to be dispensed from the fluid reservoir 901 and, thereafter, automatically close to prevent reflux into the fluid reservoir 901.

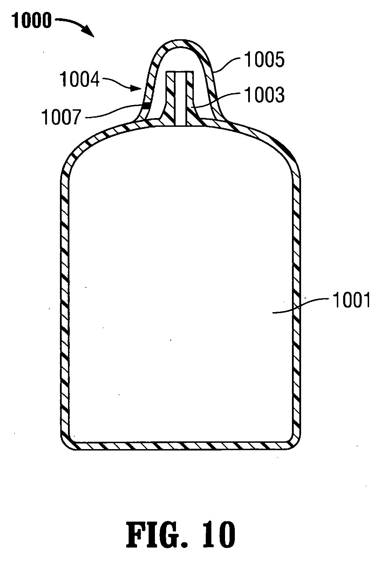

Fig. 10 is a cross-sectional view of another embodiment of the delivery device, shown generally as 1000. Delivery device 1000 includes a collapsible body 1004 that defines a fluid reservoir 1001 and includes a connector 1003. As shown in Fig. 10, the connector 1003 is welded into the body 1004. The body 1004 defines a mustard pack that may be compressed or, rolled from an end in tooth paste dispenser style to dispense fluid. The delivery device 1000 has a seal portion 1005 that covers connector 1003. The seal portion 1005 may be notched at 1107 to allow for tearing of the seal portion 1005 so that the connector 1003 may be exposed and fluid may be dispensed from the device 1000.

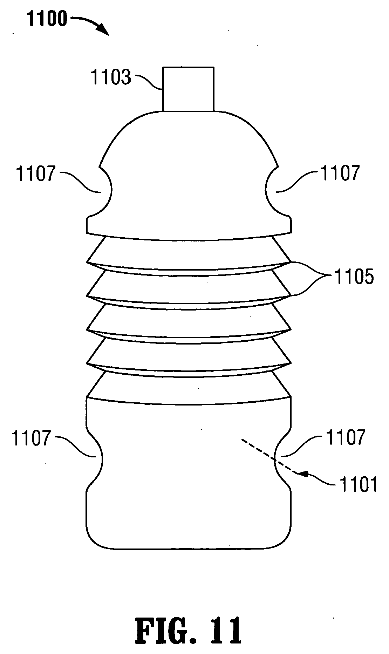

Fig. 11 is a side view of another embodiment of the delivery device shown generally as 1100. Delivery device 1100 includes a collapsible, semi-rigid body 1104 that defines a fluid reservoir 1101 attached to a connector 1103 which defines a fluid outlet. The body 1104 may include at least one section comprising one or more bellows or pleats 1105 and a section 1107 defining recesses 1107a for gripping. The body 1104 can be gripped by positioning a clinician's fingers into recesses 1107a to compress the bellows 1105 and dispense fluid from reservoir 1101. As described above, each bellow 1105 may contain to a predetermined volume of dispensed liquid, e.g. 1mL.

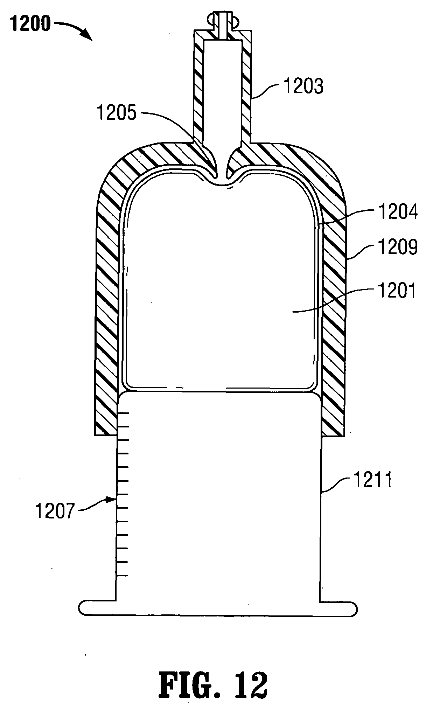

Fig. 12 is a cross-sectional view of an embodiment of the delivery device, shown generally as 1200. Delivery device 1200 includes a collapsible body 1204 that defines a fluid reservoir 1201 and a housing 1209 that includes a connector 1203. The collapsible body 1204 may comprise a fluid filled bag. The housing 1209 may include a puncturing port 1205 that is configured to puncture the body 1204 when the body 1204 is pushed against the puncturing port 1205 with a requisite or predetermined force to dispense the contents of the fluid reservoir 1201. The housing 1209 is configured to accept a plunger 1211 such that the plunger 1211 can be selectively advanced to compress the body 1204 in the housing 1209 against the puncturing port 1205 to puncture the body 1204, and to selectively force fluid out of the fluid reservoir 1201 after puncture. The plunger 1211 may have markings 1207 that indicate the amount of fluid dispensed from the fluid reservoir 1201 as the plunger 1211 is advanced into housing 1209. The plunger 1211 may be removably or permanently slidably attached to the housing 1209. If the plunger 1211 is removable, then the body 1204 that defines the fluid reservoir 1201 may be replaceable and the device 1200 may be reusable.

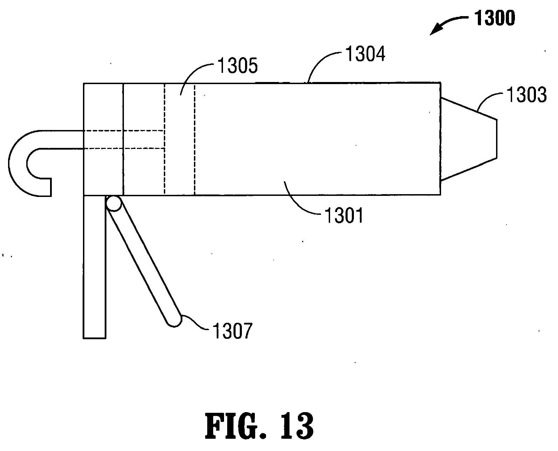

Fig. 13 is a side view of an embodiment of the delivery device, shown generally as 1300. Delivery device 1300 includes a rigid body 1304 that is dimensioned to receive a collapsible fluid reservoir 1301 and supports a connector 1303. A plunger 1305 may be slidably disposed within the rigid body 1304 such that the plunger 1305 directly contacts one end of the fluid reservoir 1301. As the plunger 1305 is advanced within rigid body 1304, the fluid reservoir 1301 is collapsed to force the fluid out through connector 1303. The plunger 1305 may be mechanically linked to lever 1307, such as by a pawl-ratchet type mechanism, such that a discrete advancement of lever 1307 advances the plunger 1305 distally toward connector 1303 a predetermined distance. Each discrete advancement of the lever 1307 may create a noticeable click that corresponds to a predetermined volume of fluid to be dispensed. The rigid body 1304 may have markings indicative of the volume of fluid being dispensed.

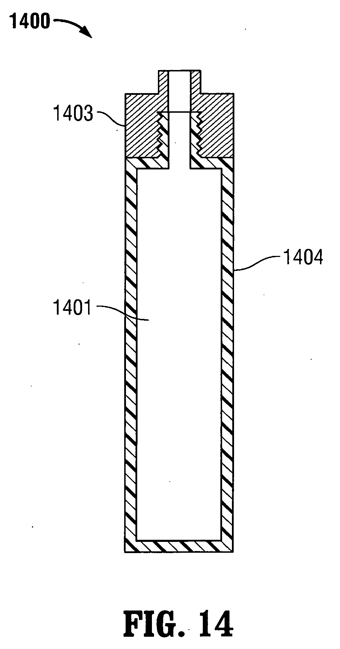

Fig. 14 is a side, cross-sectional view of an embodiment of the delivery device shown generally as 1400. Delivery device 1400 includes a deformable body 1404 that defines a fluid reservoir 1401 and includes a connector 1403. The body 1404 is comprised of a semi-rigid flexible plastic container that may be compressed in a manner similar to a plastic squeeze bottle to dispense fluid. The connector 1403 may be sealed with a pull tab seal or cap as described above that can be removed when it is desired to use the delivery device. The body 1404 may have markings to indicate the amount of fluid contained in the fluid reservoir 1401.

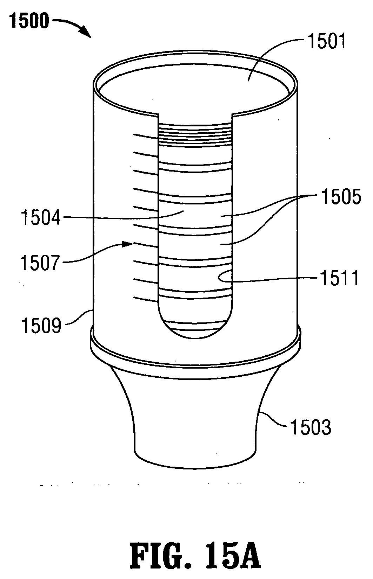

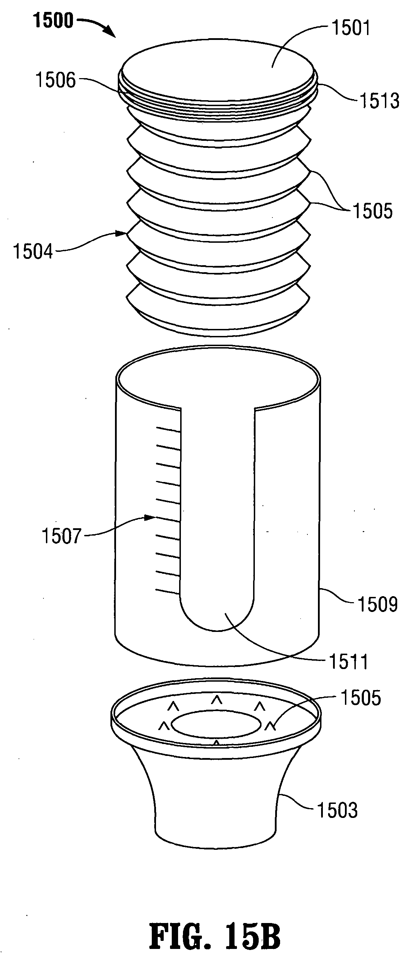

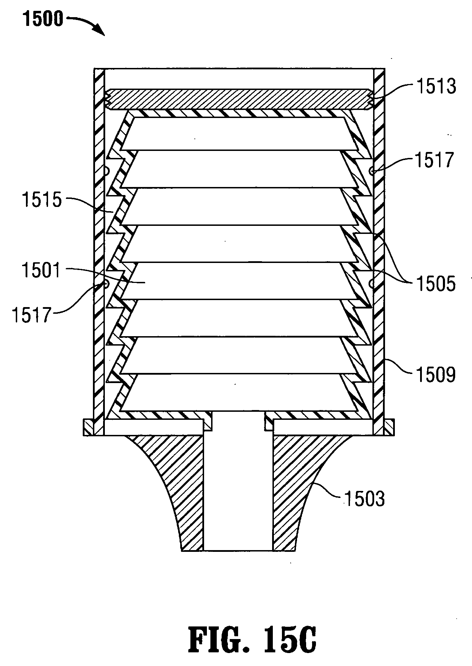

Fig. 15A is a perspective view, Fig. 15B is an exploded view, and Fig. 15C is a cross-sectional view of an embodiment of the delivery device shown generally as 1500. Delivery device 1500 includes a collapsible body 1504 that defines a fluid reservoir, a housing 1509 configured to hold the body 1504, and a connector 1503 disposed on a distal end of the housing 1509. The connector 1503 may be integrally formed with housing 1509, or releasably attachable thereto. As shown, the body 1504 includes one or more bellows 1505 as described herein. The body 1504 is removably positioned within housing 1509 such that the device 1500 may be reusable. Alternatively, the body 1504 may also be permanently attached to the housing 1509 via any suitable method. The connector 1503 may include one or more spikes 1515 that puncture the body 1504 and enter into the fluid reservoir 1501 when the body 1504 is pushed up against the spikes 1515 during compression of body 1504 in the housing 1509 to dispense fluid from body 1504.

The housing 1509 has a cutout 1511 dimensioned to receive a finger or thumb to provide access to body 1504 and facilitate compression of body 1504 to dispense fluid from the fluid reservoir 1501. The housing 1509 may have markings 1507 that correspond to the amount of fluid that is in the fluid reservoir 1501. The delivery device 1500 may also include a locking feature wherein the body 1504 supports a disc 1506 (FIG. 15C) having one or more teeth 1513 that communicate with one or more notches 1517 (FIG. 15C) formed on an inner wall of housing 1509 to lock the body 1504 in a plurality of positions as fluid is dispensed from reservoir 1501. By locking the body 1504 in compressed positions, reflux into body 1504 can be prevented. Each notch 1517 may correspond to a predetermined amount of fluid to be dispensed, such as, for example 1mL.

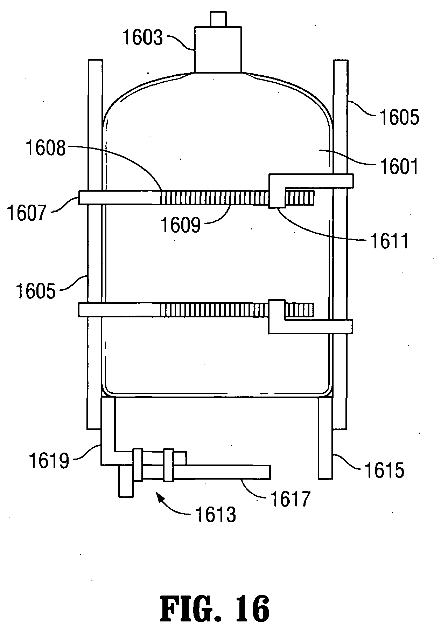

Fig. 16 is a side view of an embodiment of the delivery device, shown generally as 1600. Delivery device 1600 includes a deformable body 1604 that defines a fluid reservoir 1601 and includes a connector 1603. The body 1604 may be a flexible bag or semi-rigid flexible container that may be compressed as described herein. The delivery device 1600 may include rigid members 1605 disposed against the body 1604. The rigid members 1605 may be squeezed together to dispense fluid from the fluid reservoir 1601.

In one embodiment, the rigid members 1605 are strapped together using one or more locking members 1607. The locking members 1607 may be in the form of cable ties which include a band 1608 having notches 1609 and a receiving portion 1611 defining an opening (not shown). The band 1608 extends through the opening in receiving portion 1611 such that as the notches 1609 are pulled through receiving portion 1611, the receiving portion 1611 prevents reverse motion of the band 1608. By preventing reverse motion of band 1608 in relation to receiving portion 1611 expansion of fluid reservoir 1601 and reflux of fluid into the fluid reservoir 1604 are prevented. Each notch 1609 may correspond to a predetermined amount of dispensed fluid, such as 1mL. The locking members 1607 may have markings to indicate the amount of fluid contained in or dispensed from the fluid reservoir 1601.

Device 1600 may further include a stop mechanism 1613 including a first member 1615, a second member 1619, and a stop 1617. First member 1615 and second member 1619 may be attached to the body 1604 or rigid member 1605. As shown, stop 1617 may be slidably attached to second member 1619 and movable to a desired fixed position that corresponds to a predetermined amount of total fluid to be dispensed by device 1600. As the fluid reservoir 1601 is dispensed, stop 1617 will contact first member 1615 and prevent further dispensing of fluid.

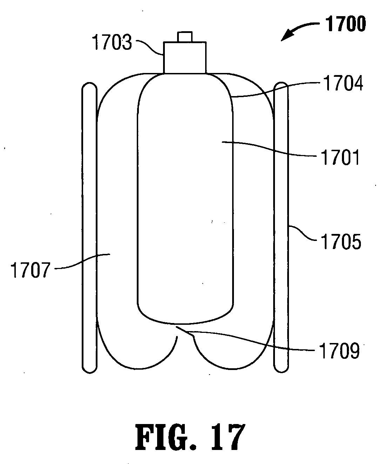

Fig. 17 is a side view of another embodiment of the delivery device, shown generally as 1700. Delivery device 1700 includes a deformable body 1704 that defines a fluid reservoir 1701 and includes a connector 1703 defining a fluid outlet. The body 1704 may be a semi-rigid flexible plastic container or a flexible bag that may be compressed as described herein. Device 1700 further includes an airbag 1707 surrounding the body 1704 such that the airbag 1707 may be selectively inflated to effect dispensing of fluid from the fluid reservoir 1701. The airbag 1707 may include check valve 1709 that allows air to selectively enter the airbag 1707 but prevents air from exiting the airbag 1707.. Airbag 1707 may also have a release valve (not shown) to selectively expel air therefrom. Similar to the embodiment of Fig. 16, the delivery device 1700 may include rigid members 1705 disposed against the body 1704 and strapped together as described above. Rigid members 1705 may be moved together to increase pressure within airbag 1707 and dispense fluid from body 1704.

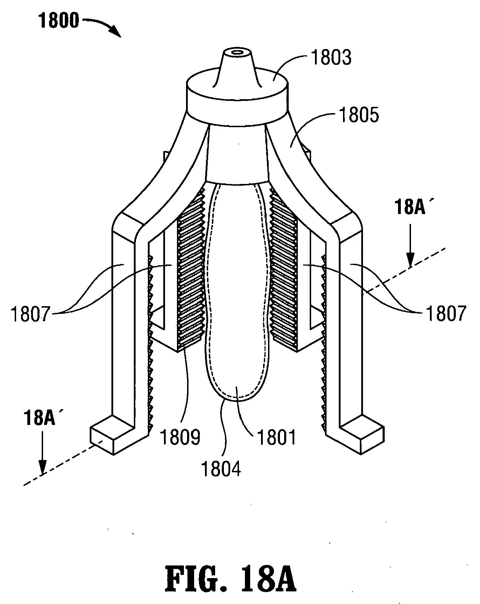

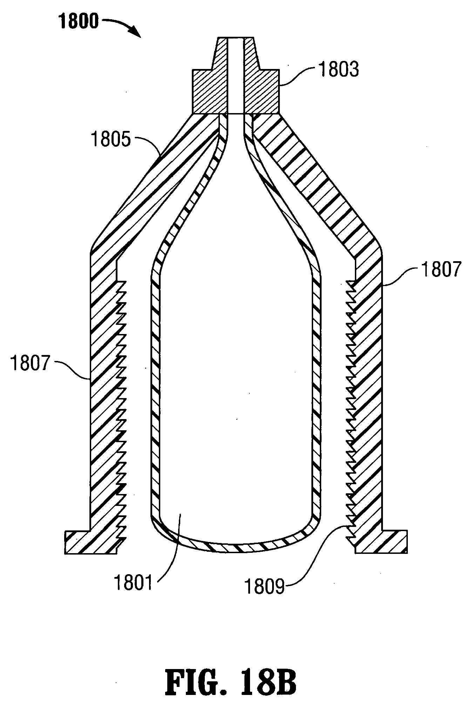

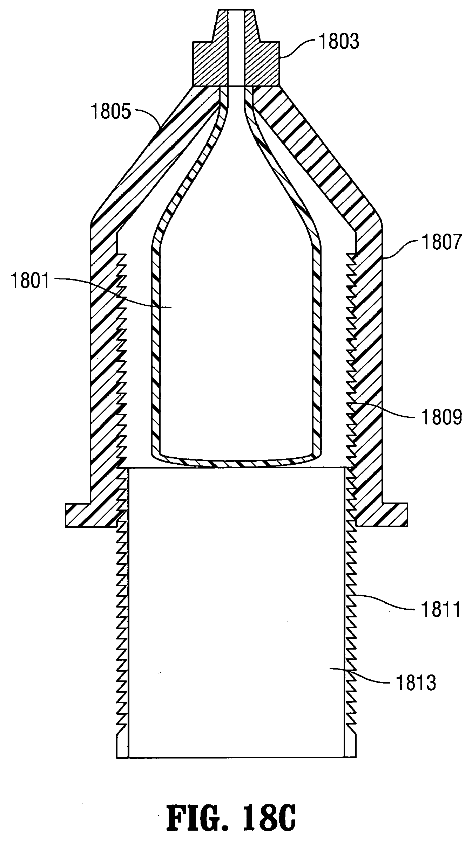

Fig. 18A is a perspective view and Figs. 18B and 18C are cross-sectional views of another embodiment of the delivery device shown generally as 1800. Delivery device 1800 includes a collapsible body 1804 that defines a fluid reservoir 1801, a housing 1805 configured to hold body 1804, and a connector 1803. The connector 1803 may form part of the housing 1805, or be attachable thereto. Referring to Fig. 18A, the body 1804 is shown in a pre-blow-molded state. Referring to Fig. 18B and 18C, the body 1804 is shown after blow molding and/or filling with fluid. The body 1804 may be provided as a cartridge which can be removably attached to housing 1805 via any suitable method including, but not limited to, a screw fit or snap fit. The body 1804 may thus be replaceable and the device 1800 may be reusable. Alternatively, the body 1804 may also be permanently attached to the housing 1805 via any suitable method.

As shown in Figs. 18A-C, the housing 1805 includes two or more arms 1807 that form a partial boundary for the body 1804. Arms 1807 form a track for a plunger 1813 (shown in Fig. 18C) to slidably interact therewith to compress the body 1804 and dispense fluid from the fluid reservoir 1801. The delivery device 1800 may also include a locking feature wherein one or more of the arms 1807 include one or more notches 1809 that communicate with one or more teeth 1811 on a plunger 1813 to progressively lock the position of the plunger 1813 in relation to the housing 1809 in progressively more advanced states. The locking feature prevents proximal movement of plunger 1813 as plunger 1813 is advanced to compress the fluid reservoir 1801 to prevent expansion of the fluid reservoir 1801 which may result in reflux of fluid into the fluid reservoir 1801. As described with respect to previous embodiments, each notch 1809 may correspond to a predetermined amount of fluid to be dispensed, such as, for example 1mL, allowing the clinician to control the amount of fluid dispensed from the fluid reservoir 1801. Plunger 1813 may have markings thereon that correspond to the volume of fluid contained in the fluid reservoir 1801. Alternatively or in conjunction therewith, the housing 1809 may also have markings to determine the amount of dispensed fluid based on the position of plunger 1813 in relation to the housing 1805.

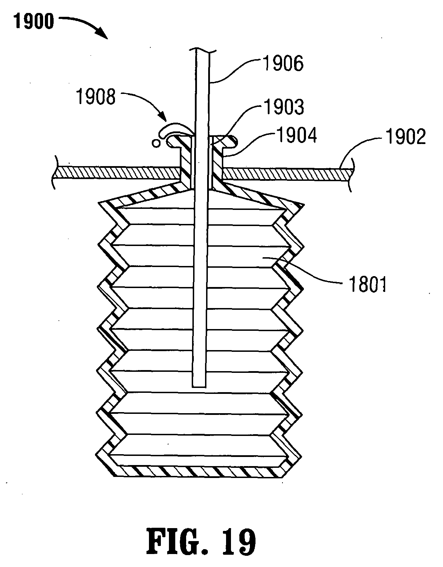

Fig. 19 is a cross-sectional view of an embodiment of a system 1900 and method for filling a body 1904 that defines a fluid reservoir 1901. Body 1904 includes a filling port 1903. The system 1900 includes a fill tube 1906 and seal clamps 1902. The fill tube 1906 may be inserted into filling port 1903 as the reservoir 1901 is held in place by seal clamps 1902 such that the reservoir 1901 can be filled with fill tube 1906. To prevent bubbles or air pockets, the reservoir 1901 may be filled until overflow fluid 1908 leaks from filling port 1903. Thereafter, the fill tube 1906 may be removed from filling port 1903, and filling port 1903 may be sealed using seal clamps 1902 or using any suitable sealing method, including melting, capping, gluing, heat sealing etc.

Persons skilled in the art will understand that the devices and methods specifically described herein and illustrated in the accompanying drawings are non-limiting exemplary embodiments. It is envisioned that the elements and features illustrated or described in connection with one exemplary embodiment may be combined with the elements and features of another without departing from the scope of the present disclosure. As well, one skilled in the art will appreciate further features and advantages of the disclosure based on the above-described embodiments. Accordingly, the disclosure is not to be limited by what has been particularly shown and described, except as indicated by the appended claims.

## Claims

1. A fluid delivery device (1500) comprising:
a body (1504) defining a fluid reservoir (1501) and a fluid outlet;
a connector (1503) including structure to connect the fluid delivery device (1500) to a medical device; and
a housing (1509) configured to receive the body (1504)
wherein the body (1504) includes one or more pleats (1505) and is collapsible to dispense fluid from the fluid reservoir (1501) through the fluid outlet and **characterized in that** the fluid delivery device (1500) further comprises a disc (1506) supported on the body (1504) within the housing (1509), the disc (1506) having one or more teeth (1513) which engage notches (1517) on the housing (1509) to lock the body (1504) in one of a plurality of positions.

2. The fluid delivery device (1500) of claim 1, wherein each of the one or more pleats (1505) contains a predetermined volume of fluid.

3. The fluid delivery device (1500) of claim 1, wherein the housing (1509) defines a cutout (1511) dimensioned to receive a clinician's finger to facilitate compression of the body (1504).

4. The fluid delivery device (1500) of claim 1, wherein the connector (1503) includes spikes (1515) positioned to puncture the body (1504).

5. The fluid delivery device (1500) of claim 1, wherein the housing (1509) includes markings (1507) to identify the volume of fluid dispensed from the reservoir (1501).

## Patentansprüche

1. Fluidabgabevorrichtung (1500), umfassend:
einen Körper (1504), der einen Fluidbehälter (1501) und einen ersten Fluidauslass definiert;
einen Verbinder (1503) mit Struktur zum Verbinden der Fluidabgabevorrichtung (1500) mit einer medizinischen Vorrichtung; und
ein Gehäuse (1509), das zum Aufnehmen des Körpers (1504) konfiguriert ist,
wobei der Körper (1504) eine oder mehrere Falten (1505) beinhaltet und zusammenschiebbar ist, um Fluid aus dem Fluidbehälter (1501) durch den Fluidauslass abzugeben, und **dadurch gekennzeichnet, dass** die Fluidabgabevorrichtung (1500) ferner eine Scheibe (1506) umfasst, die an dem Körper (1504) in dem Gehäuse (1509) getragen wird, wobei die Scheibe (1506) einen oder mehrere Zähne (1513) aufweist, die in Kerben (1517) an dem Gehäuse (1509) eingreifen, um den Körper (1504) in einer von einer Vielzahl von Positionen zu arretieren.

2. Fluidabgabevorrichtung (1500) nach Anspruch 1, wobei jede der einen oder mehreren Falten (1505) ein vorgegebenes Volumen an Fluid enthält.

3. Fluidabgabevorrichtung (1500) nach Anspruch 1, wobei das Gehäuse (1509) eine Aussparung (1511) definiert, die dazu bemessen ist, den Finger von Klinikpersonal aufzunehmen, um das Zusammendrücken des Körpers (1504) zu ermöglichen.

4. Fluidabgabevorrichtung (1500) nach Anspruch 1, wobei der Verbinder (1503) Zacken (1515) beinhaltet, die angeordnet sind, um den Körper (1504) zu durchstechen.

5. Fluidabgabevorrichtung (1500) nach Anspruch 1, wobei das Gehäuse (1509) Markierungen (1507) beinhaltet, um das Volumen an Fluid zu anzugeben, das aus dem Behälter (1501) abgegeben wurde.

## Revendications

1. Dispositif de distribution de fluide (1500) comprenant :
un corps (1504) définissant un réservoir de fluide (1501) et une sortie de fluide ;
un connecteur (1503) incluant une structure pour brancher le dispositif de distribution de fluide (1500) à un dispositif médical ; et
un logement (1509) configuré pour recevoir le corps (1504)
dans lequel le corps (1504) inclut un ou plusieurs plis (1505) et est pliable pour distribuer du fluide provenant du réservoir de fluide (1501) par la sortie de fluide et **caractérisé en ce que** le dispositif de distribution de fluide (1500) comprend en outre un disque (1506) supporté par le corps (1504) à l'intérieur du logement (1509), le disque (1506) comportant une ou plusieurs dents (1513) qui se mettent en prise avec les encoches (1517) sur le logement (1509) pour verrouiller le corps (1504) dans une d'une pluralité de positions.

2. Dispositif de distribution de fluide (1500) selon la revendication 1, dans lequel chacun du ou des plis (1505) contient un volume prédéterminé de fluide.

3. Dispositif de distribution de fluide (1500) selon la revendication 1, dans lequel le logement (1509) définit une ouverture (1511) dimensionnée pour recevoir un doigt de clinicien pour faciliter la compression du corps (1504).

4. Dispositif de distribution de fluide (1500) selon la revendication 1, dans lequel le connecteur (1503) inclut des pointes (1515) positionnées de façon à perforer le corps (1504).

5. Dispositif de distribution de fluide (1500) selon la revendication 1, dans lequel le logement (1509) inclut des marquages (1507) pour identifier le volume de fluide distribué à partir du réservoir (1501).
